# EUROPEAN PATENT SPECIFICATION

(11) **EP 0 414 396 B1**
(45) Date of publication and mention of the grant of the patent: **28.07.1993**
(21) Application number: 90308529.8
(22) Date of filing: 02.08.1990
(51) Int. Cl.: C07C 323/60, C08F 2/38

(54) **N-Hydroxyalkyl-mercaptoalkanamides**
N-Hydroxyalkyl-Mercaptoalkanamide
Mercaptoalcanamides N-hydroxyalkylés

(30) Priority: 11.08.1989 US 392491
(43) Date of publication of application: 27.02.1991
(73) Proprietor: ROHM AND HAAS COMPANY, Philadelphia Pennsylvania 19105 (US)
(72) Inventor: Gross, Andrew William, Hatborough, Pennsylvania 19040 (US); Emmons, William David, Huntingdon Valley, Pennsylvania 19006 (US)
(74) Representative: Angell, David Whilton

(56) References cited:
- EP-A- 0 128 019
- EP-A- 0 159 254
- FR-A- 2 619 811

## Description

This invention is concerned with a novel class of thiol-terminated hydroxyamide compounds which may be useful as polymerization chain transfer agents. More particularly, the invention is directed to thiol-functional hydroxyamides, preferably thiol-functional β-hydroxyalkylamides, which may be prepared by the reaction of a mercapto-functional alkylester with an alcohol-substituted amine. The thiol-terminated hydroxyamide compounds may be useful as polymerization chain transfer agents, particularly the thiol-functional hydroxyamide may be used to provide the resulting polymer or oligomer with a reactive hydroxyamide end group for subsequent preparation of block or graft copolymers.

Polymers having carboxy or anhydride groups may be effectively cured or crosslinked by treating the polymer with a β-hydroxyalkylamide or a polymer containing β-hydroxyalkyamide functionality, as disclosed in U.S.Patents: 4,076,917; 4,101,606; 4,115,637; 4,138,541; 4,727,111 and 4,801,680. Examples of suitable acid or anhydride monomers which can be incorporated into the polymer backbone and crosslinked with β-hydroxyalkylamides include: unsaturated monocarboxylic acids; such as acrylic acid, methacrylic acid and crotonic acid; unsaturated dicarboxylic acids such as maleic acid, 2-methylmaleic acid, itaconic acid, 2-methylitaconic acid and alpha,beta-methyleneglutaric acid; unsaturated anhydrides such as maleic anhydride, itaconic anhydride, acrylic anhydride and methacrylic anhydride; and amorphous carboxylic acid-group containing polyesters formed by the condensation reaction of aliphatic polyols and/or cycloaliphatic polyols with aliphatic and/or aromatic polycarboxylic acids and anhydrides.

The synthesis of β-hydroxyalkylamides and their use as crosslinkers, such as for example in the preparation of thermosetting coatings, was examined in:
β-Hydroxyalkylamides, Low Polluting Crosslinkers For Carboxyl Containing Polymers, J.Lomax and G.Swift, Journal of Coatings Technology, Vol.50, No. 643 (Aug. 1978) pages 49-55; Esterification of N(2-Hydroxyalkyl)Amides, Z.W. Wicks and N-C. Chiang, Journal of Coatings Technology, Vol.54, No. 686 (March 1982) pages 27-31; and Reaction of N-(2-Hydroxyethyl)Amido Compounds, Z.W. Wicks , M. R. Appelt and J.C. Soleim, Journal of Coatings Technology, Vol.57, No. 726, (July 1985) pages 51-61.

US-A-3148126 and American Chemical Society Chemical Abstracts vol 54 (1960) 17233-34 disclose the use of N-hydroxyethylthioglycolamide in stain and hair cosmetic applications.

None of these references, however, disclose or suggest thiol-terminated hydroxyamides or their use as chain transfer agents in polymerization reactions.

There is always a desire in the art to find new compounds which are useful as polymerisation chain transfer agents and which, in time, may supplement, or even replace, one or more conventional polymerisation chain transfer agents. Reasons for wanting to supplement or replace a conventional polymerisation chain transfer agent may be environmental, economical, specialisational and/or expansional.

It is an object of the present invention to provide a new compound which is useful as a chain transfer agent and which, in time, may satisfy one or more of the above wants.

In accordance with the present invention, there is provided novel thiol-terminated hydroxyamide compounds. preferably the compounds are thiol-terminated β-hydroxyalklamides. The thiol-terminated hydroxyamide compounds of the present invention are useful as polymerisation chain transfer agents and may be used as chain transfer agents for the preparation of hydroxyamide-functional polymers or oligomers. Such hydroxyamide-containing polymers and oligomers may be subsequently reacted with acid-containing monomers or polymers to form block and graft copolymers.

For the purpose of the present disclosure the term "oligomer" shall refer to low molecular weight polymers which can function as monomers in subsequent polymerization reactions with other monomers or polymers. These functional oligomers are also referred to in the art as "macromers" or "macromonomers". The weight average molecular weight of these oligomers is typically in the range of from as low as about 300 to as high as about 5000.

The compounds of the present invention may be prepared by the amidation of a mercapto-functional alkylester with a hydroxyamine, preferably using a molar excess of amine to ester, and may be useful for preparing graft and block copolymers.

The thiol-terminated hydroxyamide compounds of the invention are those compounds represented by the structural formula I : 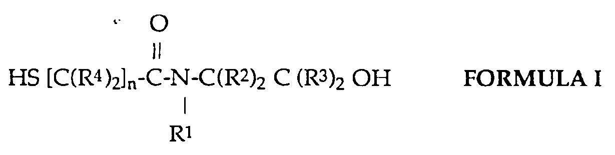
where R¹ is hydrogen, a straight or branched chain (C₁-C₅) alkyl or HO(R³)₂C(R²)₂C-;
where each R² , R³ or R⁴ radical is the same or different to any other R², R³ or R⁴ radical and is independently selected from hydrogen, or straight or branched chain (C₁-C₅)alkyl, or where one of the R2 and one of the R3 radicals may be joined together with the carbon atoms;
and where n is an integer greater than 1. Preferably n is an integer selected from the group of integers comprising greater than 1 to 20, more preferably greater than 1 to 10, even more preferably greater than 1 to 5 and most preferably 2. Where one of the R² and one of the R³ radicals are joined together with the carbon atoms, it is preferable for the R² and R³ radicals to be located on adjacent carbon atoms of Formula 1. (i.e. the R² and R³ radicals should preferably be located on carbon atoms not separated by the nitrogen atom in Formula 1).

A preferred thiol-terminated hydroxyamide of the invention has the structural formula II: 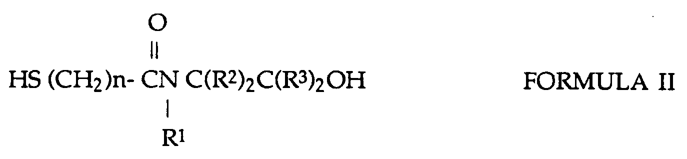
where each R¹, R² and R³ radicals , and n are as defined above, and where both R⁴ radicals in Formula I are hydrogen.

The hydroxyamine is a compound having the structural formula III: 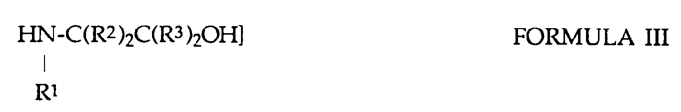
wherein R¹ is hydogen, a straight or branched chain (C₁-C₅) alkyl or HO(R³)₂C(R²)₂C-; each R² or R³ radical is the same or different to any other R² or R³ radicals and is independantly selected from hydrogen, straight or branched chain (C₁-C₅)alkyl or where one of the R² and one of the R³ radicals may be joined together with the carbon atoms. Some representative amines of this type include 2-aminoethanol; 2-methylaminoethanol; 2-ethylaminoethanol; 2-n-propylaminoethanol; 2,2′-iminodiethanol; 2-aminopropanol; 2,2′-iminodiisopropanol; 2-aminocyclohexanol; 2-aminocyclopentanol; 2-aminomethyl-2-methylethanol; 2-n-butylaminoethanol; 2-methylamino-1,2-dimethylethanol; 2-amino-2-methyl-1-propanol; 2-amino-2-methyl-1,3-propanediol; 2-amino-2-ethyl-1,3-propanediol and 2-amino-2-hydroxymethyl-1,3-propanediol.

The mercapto-functional alkylesters which may be employed are those represented by the structural formula IV:

HS [C(R^{x})₂]ₙCOO (R^{y}); FORMULA IV

where R^{y} is any alkyl, straight or branched chain, preferably (C₁-C₂₀)alkyl, more preferably (C₁-C₅)alkyl, most preferably methyl; each R^{x} is independently hydrogen, or a straight or branched chain (C₁-C₅)alkyl, preferably methyl; and n is an integer greater than 1, preferably selected from the group of integers comprising greater than 1 to 20, more preferably greater than 1 to 10, even more preferably greater than 1 to 5 and most preferably 2. When both R^{x} radicals are hydrogen and n is 2 the ester is alkyl mercaptopropionate.

Increasing the n in the mercapto-functional alkylester, or preferably using a more hydrophobic R¹ in the hydroxyamine, increases the hydrophobicity of the resultant thiol-terminated hydroxyamide. This may be useful for achieving the desired degree of either water or organic solvent solubility which may be required.

When the mercapto-functional alkylester is thioglycolate the reaction is exothermic and can be conducted at room temperatures. When the n of the mercapto-functional alkylester is 2 or greater the reaction may be conducted at temperatures in the range of from about 25 ° Centigrade to about 120° Centigrade. In either case the reaction may be conducted in the absence of a catalyst. It was found that while the time needed to complete the reaction decreased as the reaction temper ature increased within this range, it was preferable to conduct the reaction at lower reaction temperatures to increase the yield of the desired product by avoiding or minimizing the formation of undesirable by-products, such as for example those by-products formed by condensation reactions.

In addition to using milder reaction temperatures, it was found that in order to optimize the formation of the desired product a molar excess of hydroxyamine to mercapto-functional ester is preferred. It was found that if less than one equivalent of amine per equivalent of thiol is used the product yield drops dramatically (below 50%) and the presence of higher molecular weight by-products increase. It was found that it is preferred to utilize a molar excess of amine to thiol of from about 2/1 to about 3/1 and the preferred reaction temperatures in the range of from about 60 to about 80° C ( when the n of the mercapto-functional alkylester is greater than 1) to obtain a yield of product in the range of from about 70 to about 75%, and more preferably, in such instances, to utilize a molar excess of 2/1 at a temperature of 80° C.

Removal of unreacted amines and condensation by-products can be employed to increase product purity. We have found a preference for purification of the reaction product by extraction using an organic solvent, such as for example methylene chloride, rather than by vacuum distillation.

The thiol-terminated hydroxyamide may be useful as a polymerization chain transfer agent for any type of free radical initiated polymerization. The ability to terminate any free radical initiated polymer, whether prepared in water or an organic solvent, with a reactive end group may provide advantages during subsequent processing or use of the polymer so terminated over the same polymer terminated with a non reactive, conventional polymerization chain transfer agent. We have found that the thiol-terminated hydroxyamides of the invention can be employed as a chain transfer agent to prepare polymers and oligomers having weight average molecular weights as low as about 300 weight average molecular weight . In addition, the thiol-terminated hydroxyamide may be useful in chain terminating higher molecular weight polymers having weight average molecular weights on the order of 100,00 or higher. The thiol-terminated hydroxyamide is used to terminate polymerization reactions in the same manner as conventional chain transfer agents known in the art, such as for example n-dodecyl mercaptan, at the same concentrations as such conventional chain transfer agents are commonly employed , and with no special processing requirements.

By placing a hydroxy amide functional group at the end of the polymer chain the polymer produced will possess the ability to more effectively react with acid-containing polymers. This may be particularly advantageous for the preparation of thermosetting coating compositions, such as for example in acrylic or polyester powder coatings , such as, for example, to permit the coating to be formulated with a lower concentration of crosslinker to achieve the same crosslink density .

The thiol-terminated hydroxyamide is useful in the preparation of reactive oligomers. The hydroxyamide end group can be reacted with any carboxylic acid-containing compound, including carboxylic acid containing polymerizable monomers. This may be accomplished by mixing the thiol-terminated hydroxyamide with the carboxylic acid containing compound and heating the mixture to a temperature of from about 100 degrees Centigrade to about 160 degrees Centigrade, preferably from about 110 degrees to about 130 degrees Centigrade. In the case where the carboxylic acid containing compound is a polymerizable monomer, the resulting macromer, which may comprise the thiol-terminated hydroxyamide as a pendant segment on the oligomer or maaomer backbone, may then be copolymerized using conventional free radical initiation polymerization conditions with one or more copolymerizable monomers to incorporate the maaomer into the polymer backbone as pendant graft segments.

The following examples illustrate the preparation and use of the thiol-terminated hydroxyamides of the invention.

### Example 1 Preparation of Thiol-terminated Hydroxyamide

To a 500 milliliter flask was added 1 mole (117 grams) of N-butyl-N-hydroxyethylamine. The flask was cooled by placing it in an ice bath. One half mole(53 grams) of methyl thioglycolate was then added dropwise over one hour to the flask while the temperature of the flask contents was maintained at about 45 °C. The flask was then allowed to warm to room temperature over 5 hours.The contents of the flask were transferred to a separatory funnel. The flask was rinsed with 200 milliliters of methylene chloride and the rinses were added to the separatory funnel. The transferred materials were then washed with three portions (150 ml) of 5% sodium hydroxide. This served to form the thiolate anion and resulted in the extraction of the thiol into the aqueous phase. The combined basic, aqueous extracts were washed with two 50 milliliter portions of methylene chloride to remove all non-aqueous base soluble contaminants such as higher molecular weight condensation byproducts. The methylene chloride washes were discarded.. The aqueous base solution was then acidified with 6N hydrochloric acid to pH 1. This served to protonate the thiolate anion thereby reducing its water solubility and allowing its extraction into methylene chloride. The acidified aqueous phase was washed three times with 150 ml portions of methylene chloride and the combined extracts were dried by slurrying with 10 grams of anhydrous magnesium sulfate. The magnesium sulfate was removed by filtration though a glass wool plug and the methylene chloride was stripped on a Buchi rotary evaporator at 35 degrees C. at 2666 Pa (20 mm Hg). the resulting product, N-butyl-N-hydroxyethyl mercaptoacetamide (63 grams, 0.36 moles, 72% yield) was analyzed by NMR (Bruker AM-250 mHz). Silver nitrate titration showed 5.65 meq thiol/gram (theory 5.71 meq thiol/gram).

### Example 2 Preparation of N-butyl-N-hydroxyethyl-3-mercaptopropionate

To a 500 milliliter flask was added 100 grams (0.832 grams) of methyl mercaptopropionate and 195 grams(1.66 moles) of N-butylhydroxyethylamine. The reaction mixture was heated to 80 degrees Centigrade for 8 hours and then was allowed to cool to room temperature. The product, N-butyl-N-hydroxyethyl-3-mercaptopropionate, was purified as set forth in example 1. The yield of product was 70%.

### Example 3 Preparation of a hydroxyethylamide-terminated polymethyl methacrylate oligomer of molecular weight of about 2000.

To a well stirred, nitrogen flushed, flask containing 100 grams of toluene maintained at 80 degrees Centigrade was added, dropwise over 2.5 hours, in three separate feeds: 1) 300 grams of methyl methacrylate(2.99 moles) in 150 grams toluene; 2) 28.5 grams (0.149 moles) of N-butyl-N-hydroxyethyl mercaptoacetamide; and 3) 1.5 grams of 2,2-azobis-(2-methylbutyronitrile) in 10 grams of toluene as initiator. After the feeds were complete the reaction mixture was maintained at 85 degrees Centigrade for an additional hour and then was allowed to cool to room temperature. Conversion to polymer was found to be 95%.

### Example 4 Methacrylate Functionalization of the B-hydroxyethylamide-terminated polymethyl methacrylate oligomer

The polymethyl methacrylate oligomer of example 3 (330 grams, 0.149 moles) in toluene was heated to reflux with 14.1 grams (0.164 moles) of methacrylic acid along with 0.1 grams of methoxy hydroquinone as inhibitor. After 6 hours at reflux, titration of an aliquot of the reaction mixture indicated consumption of greater than 95% of the theoretical methacrylic acid. NMR analysis confirmed that almost quantitative conversion to the macromer.

### Example 5 Preparation of graft copolymers: Polybutyl acrylate with pendant methacrylate chains

A) A toluene solution of a polybutyl acrylate polymer (500 grams) containing 5% methacrylic acid was heated to reflux with 200 grams of B-hydroxyethylamide-terminated polymethyl methacrylate oligomer(Example 3). After 8 hours acid-base titration of an aliquot indicated nearly complete esterification of the polymethyl methacrylate oligomer into the polybutyl acrylate backbone.
B) To a well stirred flask containing 100 grams of toluene at 85 degrees Centigrade was added dropwise over 2.5 hours a solution of the methacrylate functional polymethyl methacrylate oligomer of example 4 (100 grams), butyl acrylate (240 grams) and methacrylic acid (10 grams). A solution of 2.5 grams of azobis-2-methylbutyronitrile in 10 grams of toluene was simultaneously fed to the reaction mixture to initiate polymerization. The reaction mixture was maintained at 85 degrees Centigrade for an additional hour and then allowed to cool to room temperature. The product was analyzed by NMR (Bruker AM-250 mHz) and by gel permeation chromatography. Both analyses demonstrated essentially complete incorporation of the polymethyl methacrylate oligomer (Example 4) into the polybutyl acrylate backbone.

## Claims

1. A thiol-terminated hydroxyamide having the structural formula : 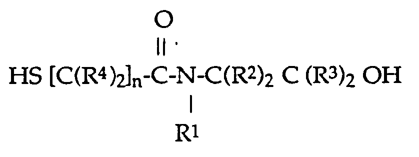 where R¹ is hydrogen, a straight or branched chain (C₁-C₅)alkyl, or HO(R³)₂C(R²)₂C-;
where each R² , R³ or R⁴ radical is the same or different to any other R² , R³ or R⁴ radical and is independently selected from hydrogen, or straight or branched chain (C₁-C₅)alkyl, or where one of the R² and one of the R³ radicals may be joined together with the carbon atoms; and where n is an integer greater than 1.

2. The thiol-terminated hydroxyamide of claim 1, wherein both R⁴ radicals are hydrogen.

3. A process for preparing a thiol-terminated hydroxyamide as claimed in claim 1 or claim 2, comprising reacting a hydroxyamine having the structural formula : 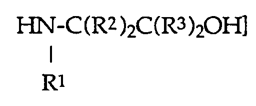 wherein R¹ is hydogen, a straight or branched chain (C₁-C₅)alkyl or HO(R³)₂C(R²)₂C-;
each R² or R³ radical is the same or different to any other R² or R³ radical and is independently selected from hydrogen, straight or branched chain (C₁-C₅) alkyl or where one of the R² and one of the R³ radicals may be joined together with the carbon atoms ;
with a mercapto-functional alkyl ester having the structural formula:
HS[C(R^{x})₂]ₙCOO (R^{y});
where R^{y} is any straight or branched chain alkyl; each R^{x} is independently hydrogen or a straight or branched chain (C₁-C₅)alkyl; and n is an integer greater than 1.

4. The process of claim 3, where the mercapto-functional alkylester is selected from the group consisting of alkylmercaptopropionate and thioglycolate.

5. The process of claim 3 or claim 4, wherein a molar excess of hydroxyamine to mercapto-functional ester is employed.

6. The process of claim 5, wherein the molar excess is from 2/1 to 3/1.

7. The process as claimed in any one of Claims 3 to 6, which process comprises the further step of purifying the reaction product using organic solvent extraction.

8. Use of a compound as claimed in Claim 1 or Claim 2 as a chain transfer agent in a free radical initiated polymerization reaction.

9. A polymer, macromer or oligomer comprising segments of the compound claimed in Claim 1 or Claim 2.

## Patentansprüche

1. Thiol-terminiertes Hydroxyamid mit der Strukturformel: 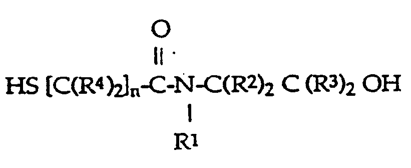 worin R¹ Wasserstoff, ein geradkettiges oder verzweigtes (C₁-C₅)Alkyl oder HO(R³)₂C(R²)₂C- ist,
worin jeder R², R³ oder R⁴ Rest gleich oder verschieden zu einem anderen R², R³ oder R⁴ Rest ist und unabhängig davon aus Wasserstoff oder einem geradkettigen oder verzweigten (C₁-C₅)Alkyl ausgewählt ist, oder worin einer der R² und einer der R³ Reste zusammen mit den C-Atomen verbunden sein können, und worin n eine ganze Zahl größer 1 ist.

2. Thiol-terminiertes Hydroxyamid nach Anspruch 1, worin beide R⁴ Reste Wasserstoff sind.

3. Verfahren zur Herstellung eines Thiol-terminierten Hydroxyamids nach Anspruch 1 oder 2, bei dem ein Hydroxyamin der Strukturformel: 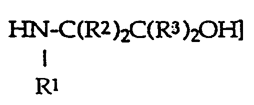 worin R¹ Wasserstoff, ein geradkettiges oder verzweigtes (C₁-C₅)Alkyl oder HO(R³)₂C(R²)₂C- ist,
worin jeder R² oder R³ Rest gleich oder verschieden zu einem anderen R² oder R³ Rest ist und unabhängig davon aus Wasserstoff oder einem geradkettigen oder verzweigten (C₁-C₅)Alkyl ausgewählt ist, oder worin einer der R² und einer der R³ Reste zusammen mit den C-Atomen verbunden sein können,
mit einem Mercapto-funktionellen Alkylester der Strukturformel:
HS[C(R^{x})₂]ₙCOO(R^{y});
umgesetzt wird, worin R^{y} ein geradkettiges oder verzweigtes Alkyl ist, jedes R^{x} unabhängig davon Wasserstoff oder ein geradkettiges oder verzweigtes (C₁-C₅)Alkyl ist, und n eine ganze Zahl größer 1 ist.

4. Verfahren nach Anspruch 3, worin der Mercapto-funktionelle Alkylester aus der Gruppe von Alkylmercaptopropionat und Thioglycolat ausgewählt ist.

5. Verfahren nach Anspruch 3 oder 4, worin ein molarer Überschuß von Hydroxyamin zu Mercapto-funktionellem Ester verwendet wird.

6. Verfahren nach Anspruch 5, worin der molare Überschuß 2/1 bis 3/1 beträgt.

7. Verfahren nach einem der Ansprüche 3 bis 6, umfassend den weiteren Schritt der Reinigung des Reaktionsproduktes unter Verwendung einer organischen Lösungsmittelertraktion.

8. Verwendung einer Verbindung nach Anspruch 1 oder 2 als Kettenübertragungsmittel in einer freie Radikaleinitiierten Polymerisationsreaktion.

9. Polymer, Macromer oder Oligomer, umfassend Segmente der Verbindung nach Anspruch 1 oder 2.

## Revendications

1. Hydroxyamide à terminaison thiol, de formule développée: 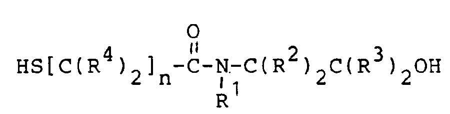 dans laquelle R¹ est un atome d'hydrogène, un groupe alkyle en C₁-C₅ à chaîne droite ou ramifiée, ou HO(R³)₂C(R²)₂C;
chaque radical R², R³ ou R⁴ est identique à ou différent de tout autre radical R², R³ ou R⁴ et est indépendamment choisi parmi un atome d'hydrogène ou un groupe alkyle en C₁-C₅ à chaîne droite ou ramifiée, ou l'un des radicaux R² et l'un des radicaux R³ peuvent être réunis avec les atomes de carbone;
et n est un nombre entier >1.

2. Hydroxyamide à terminaison thiol selon la revendication 1, dans lequel les deux radicaux R⁴ sont des atomes d'hydrogène.

3. Procédé pour la préparation d'un hydroxyamide à terminaison thiol selon la revendication 1 ou 2, comprenant la mise en réaction d'une hydroxyamine de formule développée: 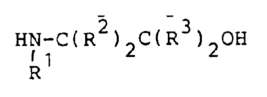 dans laquelle R¹ est un atome d'hydrogène, un groupe alkyle en C₁-C₅ à chaîne droite ou ramifiée, ou HO(R³)₂C(R²)₂C;
chaque radical R² ou R³ est identique à ou différent de tout autre radical R² ou R³ et est indépendamment choisi parmi un atome d'hydrogène ou un groupe alkyle en C₁-C₅ à chaîne droite ou ramifiée, ou l'un des radicaux R² et l'un des radicaux R³ peuvent être réunis avec les atomes de carbone;
avec un ester alkylique à fonction mercapto de formule développée:
HS[C(R^{x})₂]ₙCOO(R^{y}),
dans laquelle R^{y} est un groupe alkyle quelconque à chaîne droite ou ramifiée; chaque radical R^{x} est indépendamment un atome d'hydrogène ou un groupe alkyle en C₁-C₅ à chaîne droite ou ramifiée; et n est un nombre entier >1.

4. Procédé selon la revendication 3, dans lequel l'ester alkylique à fonction mercapto est choisi parmi un mercaptopropionate d'alkyle et un thioglycolate d'alkyle.

5. Procédé selon la revendication 3 ou 4, dans lequel on utilise un excès molaire d'hydroxyamine par rapport à l'ester à fonction mercapto.

6. Procédé selon la revendication 5, dans lequel l'excès molaire va de 2:1 à 3:1.

7. Procédé selon l'une quelconque des revendications 3 à 6, lequel procédé comprend l'étape supplémentaire de purification du produit de réaction au moyen d'une extraction par solvant organique .

8. Utilisation d'un composé tel que revendiqué dans la revendication 1 ou 2, en tant qu'agent de transfert de chaîne dans une réaction de polymérisation amorcée par des radicaux libres.

9. Polymère, macromère ou oligomère comprenant des segments du composé revendiqué dans la revendication 1 ou 2.
